Europäisches Patentamt

(19) European Patent Office (11) Publication number: **0 317 518**
**B1**
Office européen des brevets

(12) **EUROPEAN PATENT SPECIFICATION**

(45) Date of publication of the patent specification: (51) Int. Cl.⁵: **A61M 5/32**
**05.12.90**

(21) Application number: **88830402.9**

(22) Date of filing: **05.10.88**

(54) Needle protector device for syringe.

(30) Priority: **08.10.87 IT 4402387**

(43) Date of publication of application:
**24.05.89 Bulletin 89/21**

(45) Publication of the grant of the patent:
**05.12.90 Bulletin 90/49**

(84) Designated Contracting States:
**AT BE CH DE ES FR GB GR IT LI LU NL SE**

(56) References cited:
**GB-A- 2 079 607**
**US-A- 4 139 009**
**US-A- 4 664 654**
**US-A- 4 693 708**

(73) Proprietor: **Tamburini, Carlo, 4, Rue de Amicis,**
**I-61032 Fano (Pesaro)(IT)**

(72) Inventor: **Tamburini, Carlo, 4, Rue de Amicis,**
**I-61032 Fano (Pesaro)(IT)**

(74) Representative: **Fiammenghi, Carlo et al, c/o**
**Fiammenghi-Fiammenghi Via Quattro Fontane, No. 31,**
**I-00184 Roma(IT)**

ACTORUM AG

## Description

This invention relates to a device for protecting the needle of a syringe before and after the use thereof.

Syringes left in the parks, meadows, beaches and other sites frequented also by children very often cause accidental pricks with feasible transmission of deseases. Reusing the same syringe by several people is particularly dangerous. To overcome these problems US-A 4 664 654 suggests a safety device, which is positioned around the needle of a syringe. Said device comprises a spring-loaded sliding member along a guide element, which is provided with retaining means for protruding elements of the sliding member in its closed position. When the safety device is open, the tip of the needle only is exposed. In order to expose completely the needle, the user has to use both the hands, the one to maintain the sliding member against the action of the spring, the other one to press the syringe plunger. Lock means adapted to prevent the needle to be reused are not provided..

The present invention seeks to obviate the still existing problems by providing an improved device as defined in the second part of Claim 1 which, besides isolating the needle of a new syringe to keep it sterile, protects automatically the needle after being used also in case the syringe is violently thrown away in order to prevent accidental pricks with feasible infection. According to the invention the device can be rapidly released by operating a suitable safety lock, thus allowing the normal use of the syringe.

The device according to the appended claim 1 consists of a protection housing which is pushed in the forward direction by a spring or other elastic member, thus protecting the needle under normal conditions, and can be easily and manually withdrawn upon use of the syringe. When the latter is then left or thrown away the elastic member pushes the housing to protect the needle. A safety lock means is provided to prevent the protection housing from sliding along the syringe when the latter is not used.
This safety lock means can be such that reusing the syringe is very hard or impossible.

The invention will be set forth in greater detail in the following description of some embodiments thereof with reference to the annexed drawing, in which:

Fig. 1a and 2 are partially sectioned side views of a first embodiment of the protection device for a syringe under protection conditions and in a manually operated non-protected state, respectively;
Fig. 1b is a back end view of the syringe without cap of Fig. 1;
Fig. 3 shows a similar view as Figs. 1 and 2 of another embodiment of the syringe with a different safety lock means;
Figs. 4, 5 and 6 show another embodiment of the protection device with different safety lock means in partially sectioned side views showing the syringe before, during and after its use, respectively.

In the drawing the same numerals are used to indicate the same or similar parts in different embodiments. In particular, the barrel of the syringe is designated by 1, the outer end of the plunger rod by 2, the protection cap by 3 and the support of joint member of the needle by 4. A rigid housing to protect the needle is designated by 6. Such housing is normally pushed in the forward direction by an elastic member 7 in order to protect needle 5.

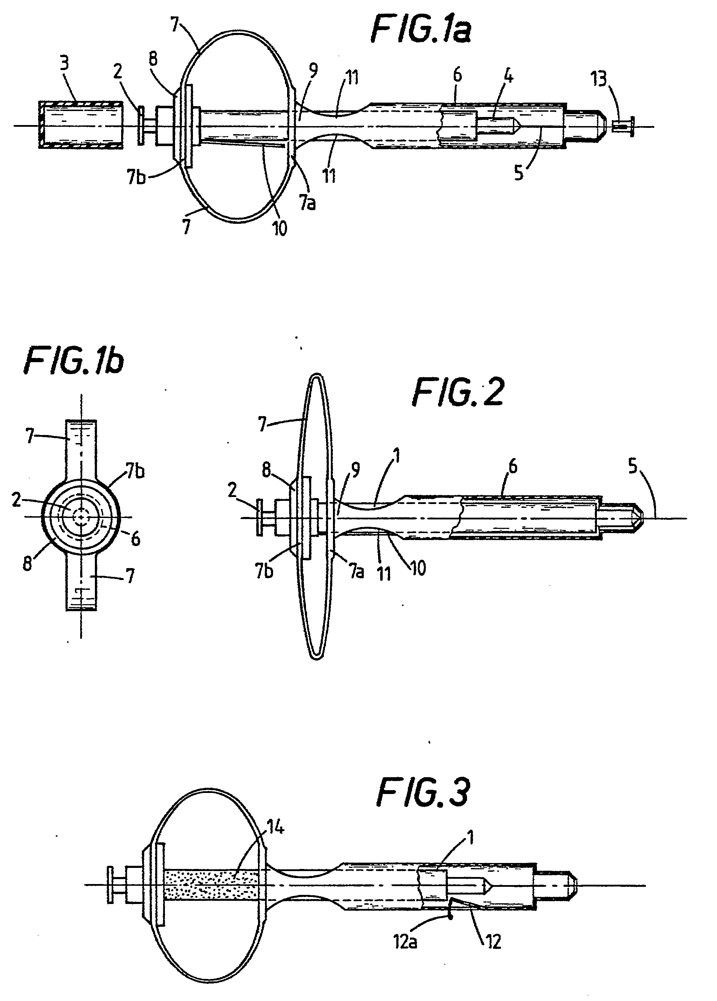

According to a first embodiment (in particular, Figs. 1a, 1b) elastic member 7 has generally the form of a ring and provides two coaxially bored and diametrally opposed expansions 7a, 7b one of which is integral with end 9 of the sliding housing, the other is integral with nut 8 at the proximal end of the barrel 1 . Elastic member 7 can be of plastic as protection housing 6 but could be replaced by a spring or any other shaped elastic member. It should perform its elastic action in the longitudinal direction of the syringe. By 10 (Fig. 1a, 1b and 2) a safety lock means is shown which is formed of an elastic blade which under rest condition takes the position of Fig. 1, thus preventing protection housing 6 to slide back. By pushing blade 10 against the barrel 1, protection housing 6 can slide back, thus overcoming the force of elastic member 7 so that needle 5 is free from protection housing. Thus, blade 10 is inserted into the gap between the barrel 1 and housing 6 or into an eventual slot provided within the housing itself. In order to allow plunger 2 of the syringe to be operated and at the same time protection housing 6 to be kept in retracted position the housing is provided with slots 11 allowing the barrel 1 to be caught by the fingers. Thus, it is possible to use the syringe with only one hand.

After use, when the syringe is left or thrown away, elastic member 7 automatically pushes forward housing 6, resilient blade 10 is released and prevent the syringe from being used again. Therefore needle 5 is completely protected by the housing, thus preventing accidental pricks.

In the embodiment of Fig. 3 the safety lock means is formed of a tongue 12 eventually cut from the protection housing and elastically bent upwards, thus abuting against the distal end of the barrel 1 and preventing the protection housing to slide back. By extracting tongue 12 through its grip member 12a and by withdrawing housing 6 through elastic member 7, the syringe can be normally used.

In order to make the grip on the barrel 1 easier the outer surface thereof can be provided with a rough area designated by 14 in the figures.

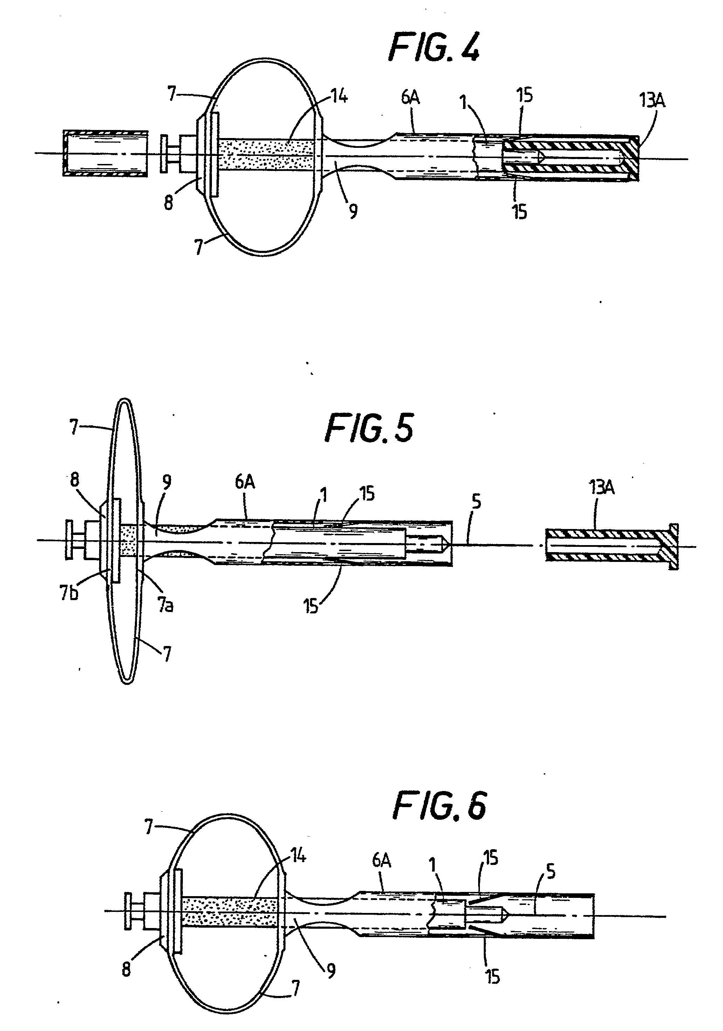

With reference to Figs. 4 and 6 there is shown another embodiment of the invention providing as safety lock means against the accidental extraction of the needle a pair of tongues 15, 15 cut into the rigid housing in opposite positions and slightly sloping inwards. The tongues have their free ends close to the joint 4 of cylinder 1 and against the outer surface of a hollow plug 13A (Fig. 4). The needle can be kept in sterile condition. The outer diameter of plug 13A is generally the same as the outer diameter of the barrel 1. Therefore, needle 5 is extracted by pressing elastic member 7 and causing the free ends of tongues 15, 15 to slide from their rest position around plug 13A to the position around the barrel 1. The plug can be then extracted (Fig. 5) and the syringe can be normally used by holding elastic member 7 pressed in the hand. After use the elastic member is released so that housing 6 protects the needle again.

Tongues 15, 15 are resiliently bent inwards, thus preventing the needle to be extracted. The syringe cannot be used anymore unless plug 13A is inserted again to spread tongues apart.

The described device with its embodiments can be applied to ordinary syringes or integrated into suitable syringes. Elastic member or spring can be adjustable. The construction could be different from that shown as an example without departing from the scope of the present invention, as defined by the following claims.

## Claims

1. Device for protecting the needle of a syringe before and after the use thereof, comprising
- a protection housing which is coaxial with the syringe and can slide along the same syringe, from a proximal position, which leaves the needle exposed, to a distal position which fully protects the needle
- an elastic means being able to perform its elastic action in the direction of the syringe axis so as to cause, under rest condition, the protection housing to assume the distal position,
- and a safety lock means adapted to prevent the elastic means to be accidentally compressed
characterized in that said protection housing, which comprises a rigid tubular element (6, 6A) and a cap mounted at its distal end, encloses the needle (5), the needle joint member (4) and a portion of the syringe barrel (1), said rigid tubular element being provided with two opposite slots (11) allowing the barrel (1) of the syringe to be suitable handled;
- and in that said elastic means is acting between the proximal end (9) of the rigid tubular element (6, 6A) and the proximal end of the barrel (1).

2. The device of claim 1, characterized in that said rigid tubular element (6) is cylindrical and has an inside diameter somewhat larger than that of both the joint member (4) and the syringe barrel (1).

3. The device of claim 1, characterized in that said rigid tubular element (6A) is a cylinder having constant diameter.

4. The device of claim 1, characterized in that said elastic means consists of an elastic ring (7) of plastic material provided with two coaxially bored diametrally opposed expansions (7a, 7b), each of them has a hole with a larger diameter than the syringe cylinder, one of said expansion being integral with the proximal end (9) of the protection housing, the other expansion being integral with a nut (8) connected to the proximal end of the barrel (1).

5. The device of claim 1, 2 and 4, characterized in that the safety lock means is a moving projection (10) generally parallel to the syringe barrel and opposing the compression of said elastic ring (7).

6. The device of claim 1, 2 and 4, characterized in that the safety lock means is a resilient tongue (12) sloped to the interior of the rigid tubular element (6) and abuting against the forward end of the syringe barrel, said tongue being also provided with a grip member (12a) projecting outwards through an opening of the rigid tubular element (6) the operation of which allows the tubular element (6) to slide along the syringe cylinder.

7. The device of claims 1, 3 and 4, characterized in that the rigid tubular element (6A) is cut to form two tongues (15, 15) diametrally opposed to each other and resiliently bent to the interior of the rigid tubular element (6A) and against a removable hollow plug (13A) surrounding the joint member (4) of the needle and the needle (5) itself, so that said plug (13A) being removed, the tongues (15, 15) engage the distal end of the barrel, thus preventing the needle to be extracted after the use of the syringe.

8. A device according to any of the preceeding claims, wherein the outer surface of cylinder (1) is rough at the grip portion (14) thereof.

## Patentansprüche

1. Vorrichtung zum Schützen der Nadel einer Injektionsspritze vor und nach dem Gebrauch, mit:
- einem Schutzgehäuse, das koaxial mit der Injektionsspritze ist und entlang der Spritze von einer proximalen Stellung, welche die Nadel ungeschützt läßt, zu einer distalen Stellung, in der die Nadel vollkommen geschützt ist, verschiebbar ist;
- einem elastischen Mittel, das in der Lage ist, seine elastische Wirkung in Richtung der Achse der Injektionsspritze auszuüben, sodaß das Schutzgehäuse unter Ruhebedingungen die distale Stellung einnimmt,
- und einem Sicherheitsverriegelungsmittel, das geeignet ist zu verhindern, daß das elastische Mittel ungewollt zusammengedrückt wird, dadurch gekennzeichnet, daß das Schutzgehäuse, welches ein starres röhrenförmiges Element (6, 6A) und eine an seinem distalen Ende angebrachte Kappe umfaßt, die Nadel (5), das Nadelansatzstück und einen Abschnitt des Spritzenzylinders (1) umschließt, wobei das starre röhrenförmige Element mit zwei gegenüberliegenden Schlitzen (11) versehen ist, die eine geeignete Handhabung des Spritzenzylinders (1) zulassen; und daß das elastische Mittel zwischen dem proximalen Ende (9) des starren röhrenförmigen Elementes (6, 6A) und dem proximalen Ende des Spritzenzylinders (1) wirkt.

2. Vorrichtung nach Anspruch 1, dadurch gekennzeichnet, daß das starre röhrenförmige Element (6) zylindrisch ist und einen Innendurchmesser hat, der etwas größer ist, sowohl als der des Ansatzstückes (4) als auch der des Spritzenzylinders (1).

3. Vorrichtung nach Anspruch 1, dadurch gekennzeichnet, daß das starre röhrenförmige Element (6A) ein Zylinder mit konstantem Durchmesser ist.

4. Vorrichtung nach Anspruch 1, dadurch gekennzeichnet, daß das elastische Mittel aus einem elastischen Ring (7) aus Kunststoffmaterial besteht, der mit zwei mit koaxialen Bohrungen versehenen diametral entgegengesetzten Ausweitungen (7a, 7b) versehen ist, wovon jede ein Loch mit einem größeren Durchmesser als der des Spritzenzylinder aufweist, wobei eine der Ausweitungen einstückig mit dem proximalen Ende (9) des Schutzgehäuses, und die andere Ausweitung einstückig mit einer mit dem proximalen Ende des Spritzenzylinders (1) verbundenen Mutter (8) ausgebildet ist.

5. Einrichtung nach Anspruch 1, 2 und 4, dadurch gekennzeichnet, daß das Sicherheitsverriegelungsmittel eine bewegliche Verlängerung (10) ist, die im allgemeinen parallel zum Spritzenzylinder verläuft und der Kompression des elastischen Ringes (7) entgegenwirkt.

6. Einrichtung nach Anspruch 1, 2 und 4, dadurch gekennzeichnet, daß das Sicherheitsverriegelungsmittel eine federnde Zunge (12) ist, die zum Inneren des starren röhrenförmigen Elementes (6) abfällt und am Vorderende des Spritzenzylinders anstößt, wobei die Zunge auch mit einem Griffelement (12a) versehen ist, welches durch eine Öffnung des starren röhrenförmigen Elementes (6) nach außen ragt und dessen Betätigung es dem röhrenförmigen Element (6) ermöglicht, den Spritzenzylinder entlangzugleiten.

7. Einrichtung nach Anspruch 1, 3 und 4, dadurch gekennzeichnet, daß das starre röhrenförmige Element (6A) so geteilt ist, daß sich zwei Zungen (15, 15) bilden, die einander diametral entgegengesetzt sind und federnd zum Inneren des starren röhrenförmigen Elementes (6A) und gegen einen abnehmbaren hohlen Stopfen (13A) gebogen sind, der das Ansatzstück (4) der Nadel (5) und die Nadel (5) selbst umgibt, sodaß die Zungen (15, 15) bei Abnehmen des Stopfens mit dem distalen Ende des Spritzenzylinders in Eingriff treten, wodurch verhindert wird, daß die Nadel nach Gebrauch der Spritze herausgezogen wird.

8. Einrichtung gemäß irgendeinem der vorangehenden Ansprüche, wobei die Außenfläche des Zylinders (1) an dessen Greifabschnitt (14) rauh ist.

## Revendications

1. Dispositif de protection d'une aiguille de seringue avant et après son usage, comprenant
- un étui de protection qui est coaxial avec la seringue et qui peut coulisser le long de la même seringue, entre une position proximale qui laisse l'aiguille dégagée, et une position distale qui protège complètement l'aiguille, un moyen élastique susceptible d'exercer son action élastique dans la direction de l'axe de la seringue afin de forcer, à l'état de repos, l'étui de protection à prendre la position distale,
- et un moyen de verrouillage de sécurité conçu pour empêcher le moyen élastique d'être comprimé accidentellement,
caractérisé en ce que ledit étui de protection qui comprend un élément tubulaire rigide (6, 6A) et un capuchon monté à son extrémité distale, enferme l'aiguille (5), l'élément d'assemblage de l'aiguille (4) et une partie du corps de la seringue (1), ledit élément tubulaire rigide portant deux fentes (11) placées face à face permettant de manipuler convenablement le corps (1) de la seringue,

- et en ce que ledit moyen élastique agit entre l'extrémité proximale (9) de l'élément tubulaire rigide (6, 6A) et l'extrémité proximale du corps (1).

2. Dispositif selon la revendication 1, caractérisé en ce que ledit élément tubulaire rigide (6) est cylindrique et en ce que son diamètre intérieur est légèrement supérieur à celui de l'élément d'assemblage (4) et du corps de la seringue (1).

3. Dispositif selon la revendication 1, caractérisé en ce que ledit élément tubulaire rigide (6A) est un cylindre de diamètre constant.

4. Dispositif selon la revendication 1, caractérisé en ce que ledit moyen élastique est constitué d'une bague élastique (7) en matière plastique portant deux prolongements coaxiaux diamétralement opposés (7a, 7b), percés chacun d'un orifice dont le diamètre est supérieur à celui du corps de la seringue, l'un desdits prolongements faisant partie intégrante de l'extrémité proximale (9) de l'étui de protection, et l'autre prolongement faisant partie intégrante d'un écrou (8) relié à l'extrémité proximale du corps (1).

5. Dispositif selon les revendications 1, 2 et 4, caractérisé en ce que le moyen de verrouillage de sécurité est une pièce en saillie mobile (10) généralement parallèle au corps de la seringue s'opposant à la compression de ladite bague élastique (7).

6. Dispositif selon les revendicatoins 1, 2 et 4, caractérisé en ce que le moyen de verrouillage de sécurité est une languette élastique (12) en pente vers l'intérieur de l'élément tubulaire rigide (6) et venant buter contre l'extrémité avant du corps de la seringue, ladite languette étant aussi équipée d'un élément d'agrafe (12a) faisant saillie vers l'extérieur à travers une ouverture de l'élément tubulaire rigide (6) dont le fonctionnement permet à l'élément tubulaire (6) de coulisser le long du corps de la seringue.

7. Dispositif selon les revendications 1, 3 et 4, caractérisé en ce que l'élément tubulaire rigide (6A) est découpé pour former deux languettes (15, 15) diamétralement opposées entre elles et coudées de manière élastique vers l'intérieur de l'élément tubulaire rigide (6A) et contre un bouchon creux amovible (13A) entourant l'élément d'assemblage (4) de l'aiguille et l'aiguille (5) elle-même, de façon qu'une fois ledit bouchon (13A) retiré, les languettes (15, 15) viennent en prise avec l'extrémité distale du crops de la seringue, en empêchant ainsi l'aiguille d'être extraite après l'utilisation de la seringue.

8. Dispositif selon l'une quelconque des revendications précédentes, dans lequel la surface extérieure du corps (1) est rugueuse dans la partie (14) qui sert à la tenir.

EP 0 317 518 B1

*FIG.1a*

*FIG.1b*

*FIG.2*

*FIG.3*

## FIG. 4

## FIG. 5

## FIG. 6